(19) Europäisches Patentamt
European Patent Office
Office européen des brevets

(11) Veröffentlichungsnummer: **0 352 652 B1**

(12) # EUROPÄISCHE PATENTSCHRIFT

(45) Veröffentlichungstag der Patentschrift: **25.01.95**

(51) Int. Cl.[6]: **A61K 31/415**, A61K 31/395

(21) Anmeldenummer: **89113425.6**

(22) Anmeldetag: **21.07.89**

Die Akte enthält technische Angaben, die nach dem Eingang der Anmeldung eingereicht wurden und die nicht in dieser Patentschrift enthalten sind.

(54) **Verwendung von Imexon als Immunsuppressivum.**

(30) Priorität: **28.07.88 DE 3825667**

(43) Veröffentlichungstag der Anmeldung:
**31.01.90 Patentblatt 90/05**

(45) Bekanntmachung des Hinweises auf die Patenterteilung:
**25.01.95 Patentblatt 95/04**

(84) Benannte Vertragsstaaten:
**AT BE CH DE ES FR GB GR IT LI LU NL SE**

(56) Entgegenhaltungen:
**DE-A- 2 528 460**
**FR-A- 2 399 840**

**IMMUNOL. SER., Band 25, 1984; U.F.BICKER, Seiten 447-473.**

**FORTSCHR. MED., Band 105, 1987; U.BICKER et al., Seiten 509-512.**

**ICRS, Band 5, 1977; U.BICKER et al., Seiten 681-684.**

**FORTSCHR. MED., Band 96, Nr. 12, 1978; U.BICKER, Seiten 681-684.**

(73) Patentinhaber: **BOEHRINGER MANNHEIM GMBH**

**D-68298 Mannheim (DE)**

(72) Erfinder: **Herrmann, Dieter, Dr.med.**
**An der Neckarspitze 13**
**D-6900 Heidelberg (DE)**
Erfinder: **Haag, Rainer, Dr.rer.nat.**
**Stahlbühlring 1**
**D-6802 Ladenburg (DE)**
Erfinder: **Bosies, Elmar, Dr.phil.nat.**
**Delpstrasse 11**
**D-6940 Weinheim (DE)**
Erfinder: **Bicker, Uwe, Prof. Dr.**
**Frankenstrasse 25**
**D-6140 Bensheim (DE)**
Erfinder: **Kampe, Wolfgang, Dr.rer.nat.**
**Ahornstrasse 42**
**D-6805 Heddesheim (DE)**

**EP 0 352 652 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

IMMUNOPHARMACOLOGY & IMMUNOTOXICOLOGY, Band 12, Nr. 1, 1990, Marcel Dekker,Inc.; M.A.CHIRIGOS et al., Seiten 1-21.

CANCER TREATMENT SYMPOSIA, Band 1, 1985; M.MIKSCHE et al., Seiten 27-35.

PROCEEDINGS OF AACR, Band 26, März 1985; Seite 281, Nr. 1109.

JOURNAL OF BIOLOGICAL RESPONSE MODIFIERS, Band 5, 1986, Raven Press, NY (US);Y.Z.PATT et al., Seiten 263-269.

## Beschreibung

Die vorliegende Erfindung betrifft die Verwendung von Imexon zur Herstellung von Arzneimitteln mit immunsuppressiver Wirkung.

Insbesondere bezieht sich die Erfindung auf die Verwendung von Imexon zur Herstellung von Arzneimitteln zur Behandlung von Autoimmunerkrankungen, B-Zell oder Plasmazell-Neoplasien, lymphoblastischen Lymphomen und Abstoßungsreaktionen nach Gewebe- oder Organtransplantationen. Allgemein kann Imexon bei der Behandlung von Krankheiten eingesetzt werden, bei denen eine pathophysiologisch erhöhte B-Lymphozyten-Proliferation oder B-Lymphozyten-Aktivierung zu beobachten ist.

Außerdem ist Gegenstand der vorliegenden Erfindung die Verwendung als Kombinationspräparat des Wirkstoffs Imexon und eines weiteren wirksamen Chemotherapeutikums, beispielsweise eines zusätzlichen Immunsuppressivums oder einer antiretroviral wirksamen Verbindung.

Die systematische Bezeichnung von Imexon, dessen Strukturformel in Abb. 1 dargestellt ist, lautet 4-Imino-1,3-diazabicyclo-(3.1.0)-hexan-2-on.

Abb. 1

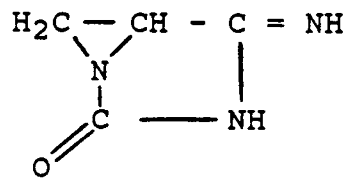

Imexon ist bezüglich seiner Struktur mit keiner anderen in der Therapie bisher eingesetzten Wirksubstanz vergleichbar. Auch die überraschenderweise gefundene bevorzugte Wirkung auf B-Lymphozyten zeigt keine Parallelen zu anderen bisher bekannten immunsuppressiv wirkenden Verbindungen.

Imexon und Verfahren zu dessen Herstellung sind aus US-A 4,083,987 bekannt. Die Verbindung ist dort als cancerostatisch wirksames Therapeutikum beschrieben, das immunstimulierende Eigenschaften aufweist. Die cancerostatische Wirkung wurde anhand der Hemmung des Tumorwachstums von Walker-Sarkoma 256 nach Applikation von Imexon bei Ratten nachgewiesen. Die immunstimulierende Wirkung ließ sich aus Versuchen ableiten, in denen ein Anstieg der Leukozyten sowie ein Anstieg der Zahl der Antikörper bildenden Milzzellen nach Applikation von Imexon beobachtet werden konnte. Die sich daraus ergebende pharmakologische Bedeutung von Imexon wird gemäß dieser US-Patentschrift darin gesehen, daß Imexon das Wachstum der sich rasch teilenden Krebszellen so stark beeinträchtigt, daß unter Umständen eine Rückbildung der Tumore möglich ist. Gemäß US-A 4,083,987 liegt die vorteilhafte Wirkung von Imexon in der gleichzeitig mit den cancerostatischen Wirkung einhergehenden Stärkung des an sich geschwächten körpereigenen Immunabwehrsystems.

Von U. Bicker et al. wird in ICRS, Band 5 (1977), Seite 428 Imexon ebenfalls als cancerostatisch wirksames Therapeutikum beschrieben, das immunstimulierende Eigenschaften aufweist.

Allgemein sind aus dem Stand der Technik Immunsuppressiva als solche schon seit längerer Zeit bekannt (Pharmazie unserer Zeit 1, 2-8 (1972) und 12, 20-29 (1983)). Der in diesem Zusammenhang verwendete Ausdruck "Immunsuppression" bezeichnet dabei im allgemeinen verschiedene Arten der unspezifischen Unterdrückung der Immunantwort, z.B. mit Hilfe von Antiseren, ionisierenden Strahlen oder speziellen Therapeutika.

Die Verwendung von immunsuppressiv wirkenden Chemotherapeutika kann Anwendung finden nach der Transplantation von Geweben oder Organen und bei der Therapie von Autoimmunerkrankungen. Sie hemmen die Proliferation von Lymphozyten durch direkten oder indirekten Eingriff in die DNA- und RNA-Synthese. Zu dieser Klasse von Verbindungen gehören Cyclosporine, Folsäure-Antagonisten, Purin-Analoga, alkylierende Substanzen wie Cyclophosphamid und bestimmte Corticosteroide. Der Nachteil dieser bisher eingesetzten Immunsuppressiva ist jedoch die in erhöhtem Maße zu beobachtende Infektionsanfälligkeit des behandelten Organismus, da das gesamte körpereigene Immunsystem geschwächt und sowohl die humorale als auch die zelluläre Immunantwort unterdrückt werden.

Die bisher bekannte artifiziell induzierte Immunsuppression konnte auf verschiedene Art und Weise erreicht werden: durch Verabreichung von Antigenen, Verabreichung von spezifischen Antisera oder Antikörpern, Verwendung von anderen biologischen Reagenzien, wie z.B. Antilymphozyten-Antisera, durch Verwendung von immunsuppressiv wirksamen Verbindungen, durch Radiation oder durch chirurgische Entfernung von lymphoidem Gewebe.

Die immunsuppressiven Eigenschaften von den meisten derzeit bekannten Immunsuppressiva, wie z.B. Zytostatika und Corticosteroiden, sind dosisabhängig aber unselektiv, d.h. sie wirken auf alle immunkompetenten Zellen. Diese Verbindungen hemmen sowohl die humorale als auch zelluläre Immunantwort auf eine Vielzahl von Antigen und wirken unspezifisch auf T- oder B-Lymphozyten. Auch Cyclosporin A, das z.Z. selektivste Medikament, supprimiert nicht nur die Proliferation von T-Lymphozyten, sondern auch Immunprozesse, die nicht T-Zell-abhängig sind.

Es besteht daher sehr großes Interesse an Immunsuppressiva, die spezifisch mit pathologisch verstärkten bzw. erhöhten Immunmechanismen interferieren, ohne jedoch die im Körper normal ablaufenden Immunreaktionen zu beeinflussen.

Lediglich Ciamexon, ein 2-Cyan-Aziridin, wird bisher in der Literatur (Fortschritte der Medizin, Band 105, 1987, Seiten 509 - 512) als eine Verbindung beschrieben, die bevorzugt antikörpervermittelte Immunreaktionen unterdrückt und T-zellvertmittelte Immunreaktionen nicht supprimiert. Daneben beschreibt Bicker in Immunol. Ser., Band 25 (1984), Seiten 447 - 473 2-Cyan-Aziridine als Immunmodulatoren, deren Immunmodulation darauf beruht, daß sie bei gleichzeitiger Stimulierung der Suppressor-T-Zellen die Helfer-T-Zell-Aktivität hemmen.

Der Erfindung lag daher die Aufgabe zugrunde, ein weiteres selektiv wirkendes immunsuppressives Mittel zur Verfügung zu stellen.

Es wurde nun überraschenderweise gefunden, daß Imexon diese Aufgabe löst und als vorteilhaftes Immunsuppressivum eingesetzt werden kann. Es supprimiert spezifisch die B-Zell-Proliferation oder B-Zell-Aktivierung. Es kann vorteilhaft bei der Behandlung von allen Krankheiten eingesetzt werden, bei denen eine polyklonale Aktivierung oder Proliferation von B-Zellen von pathophysiologischer, symptomatischer oder klinischer Relevanz ist.

In diesem Sinne kommt beispielhaft die Behandlung von folgenden Krankheiten in Frage: Autoimmunerkrankungen, wie Rheumatoide Arthritis, Diabetes mellitus Typ I, Psoriasis, Lupus Systemicus Erythematosus; Abstoßungsreaktionen nach Gewebe oder Organtransplantationen z.B. von Haut, Knochenmark und Nieren; B-Zell-Leukämien und Lymphome, wie z.B. chronisch lymphatische Leukämie, lymphoblastische Lymphome (z.B. Burkitt-Lymphom etc.) oder B-Zell/Plasmazell-Neoplasien, wie z.B. Plasmazytom (multiples Myelom).

Als Autoimmunerkrankungen werden in der Literatur im allgemeinen solche Erkrankungen bezeichnet, bei denen die Bildung von Autoantikörpern pathogenetische Bedeutung haben. Diese Autoantikörper richten sich gegen körpereigene Antigene und bewirken somit eine Zerstörung von körpereigenen Organen, Zellen oder Proteinen. Diese krankhafte Überreaktion des Immunsystems gilt es mit spezifisch wirkenden Immunsuppressiva zu unterdrücken.

Außerdem wurde überraschenderweise gefunden, daß Imexon die Proliferation von B-Lymphozyten dosisabhängig hemmt.

Imexon kann direkt selbst oder in Form seiner physiologisch verträglichen Additionssalze eingesetzt werden.

Im Sinne der vorliegenden Erfindung soll der Ausdruck "Immunsuppression" im allgemeinen alle Aspekte der natürlicherweise induzierten immunologischen Nicht-Ansprechbarkeit, der artifiziell induzierten Nicht-Ansprechbarkeit und der pathologisch induzierten Toleranz eines Individuums auf Auto- oder Fremdantigene beinhalten.

Die immunsuppressive Wirkung von Imexon konnte anhand der Hemmung der Proliferation von humanen B-Lymphozyten nachgewiesen werden, wobei die Proliferation experimentell durch den B-Zell-wachstumsfaktor (B-cell-growth factor = BCGF) induziert wird.

Weiterhin konnten die pharmakologischen Eigenschaften von Imexon durch Concanavalin A (ConA)-induzierte Proliferation muriner Splenozyten (LTT), durch Phythämagglutinin (PHA)-induzierte Proliferation humaner Lymphozyten sowie durch den Tumorwachstumsinhitionsassay (TGI) charakterisiert werden.

Um ruhende B-Zellen zur Proliferation anzuregen, sind zwei Signale erforderlich. Das erste Signal ist ein Aktivierungssignal, das durch ein Antigen oder anti- $\mu$ vermittelt wird. Die Übertragung dieses Aktivierungssignals hat schließlich zur Folge, daß auf der B-Zell-Oberfläche Rezeptoren für den B-cell-growth-factor (BCGF) exprimiert werden. Der BCGF ist ein lösliches, von T-Zellen sezerniertes Lymphokin mit einem Molekulargewicht von 17.000 -18.000 D. Die Expression von BCGF-Rezeptoren auf der B-Zelle ermöglicht dieser auf das Proliferationssignal von BCGF zu antworten. Normalerweise werden B-Zellen durch diese zwei Signalprozesse vom Ruhezustand in die proliferative Phase überführt.

Imexon supprimiert nun diesen Vorgang insofern spezifisch, als die Concanavalin A (ConA)- und Phytohämagglutinin (PHA)-induzierte Lymphozytenproliferation sowie die spontane Proliferation von Methylcholanthren-induzierten Fibrosarkomzellen (MethA) nicht oder nur bei 10-30facher höheren Konzentrationen beeinflußt werden.

Der Einfluß von Imexon auf die spontane Entstehung von Lymphomen und die Synthese von antinukleären Autoantikörper bei der Maus (Beispiel 5) belegt die Wirksamkeit an einem Tiermodell für Autoimmunerkrankungen.

Imexon kann auch als Kombinationspräparat mit anderen Immunsuppressiva, wie z.B. Cyclosporin A, Ciamexon oder Azathioprin sowie antiretroviral aktiven Substanzen, wie z.B. Azidothymidin (AZT) verwendet werden.

Ebenso ist die Kombination von Imexon mit Cytostatika möglich, wie z.B. mit cis-Platin-Komplexen, beispielsweise cis-Diaminodichloro-Platin, oder mit Adriamycin, Cyclophosphamid, Vincristin, Tamoxifen, Methotrexat oder 5-Fluorouracil, etc. In diesem Zusammenhang ist die Verwendung von derartigen Kombinationspräparaten im Anschluß an eine Plasmapharese zur Kontrolle von Autoimmunerkrankungen von besonderem Interesse.

Bei der Anwendung der Kombinationstherapie ist es möglich, die Wirkstoffe in einer sogenannten fixen Kombination, d.h. in einer einzigen pharmazeutischen Formulierung zu verabreichen, in der beide Wirkstoffe gleichzeitig vorhanden sind, oder eine sog. freie Kombination zu wählen, bei der die Wirkstoffe in Form von pharmazeutischen Formulierungen gleichzeitig oder aber auch nacheinander in individuell wählbaren Dosierungsverhältnissen appliziert werden.

Zur Herstellung pharmazeutischer Mittel wird Imexon in an sich bekannter Weise mit geeigneten pharmazeutischen Trägersubstanzen gemischt, gegebenenfalls granuliert und beispielsweise zu Tabletten oder Drageekernen verpreßt. Ebenso ist eine Abfüllung der Mischung in Steckkapseln möglich. Unter Zugabe entsprechender Hilfsstoffe kann auch eine Lösung oder Suspension in Wasser, Öl (z.B. Olivenöl) oder hochmolekularen Polymeren (z.B. Polyethylenglykol) hergestellt und zu Injektionslösungen, Weichgelantiekapseln, Saft oder Tropfen verabreicht werden.

Als feste Trägerstoffe können z.B. Stärken bzw. Stärkederivate, Zucker, Zuckeralkohole, Cellulosen bzw. Cellulosederivate, Tenside, Talkum, hochdisperse Kieselsäuren, hochmolekulare Fettsäuren oder deren Salze, Gelatine, Agar-Agar, Kalziumphosphat, tierische oder pflanzliche Fette oder Wachse und feste hochmolekulare Polymere (wie Polyethylenglykole oder Polyvinylpyrrolidone) Verwendung finden. Für orale Applikationen geeignete Zubereitungen können gewünschtenfalls Geschmacks- und Süßstoffe enthalten.

Die Dosierung des Wirkstoffes Imexon hängt vom Alter und Geschlecht des Individuums sowie von der Art der zu behandelnden Indikation ab.

Grundsätzlich kann davon ausgegangen werden, daß 0.1-100 mg Imexon pro kg Körpergewicht täglich oral, intra venös, subkutan oder intramuskular appliziert werden können. Bevorzugt sind jedoch Mengen von 5-50 mg/kg Körpergewicht, insbesondere 5-20 mg/kg. Die Wirkstoffmengen können 1 bis 3 mal täglich verabreicht werden.

Die spezifisch immunsuppressive Wirkung von Imexon wird anhand der folgenden Beispiele belegt:

Beispiel 1

BCGF-abhängige Proliferation humaner B-Lymphozyten

Die Anreicherung von peripheren humanen B-Zellen und der BCGF-Proliferationsassaywurden wie folgt durchgeführt (vgl. a. Eur. J. Immun. 16, 350 (1986)):
Angereicherte humane B-Lymphozyten werden zweimal gewaschen mit komplettiertem RPMI 1640 Medium (Streptomycin/Penicillin, L-Glutamin, 2-Mercaptoethanol, FCS) und auf 3 x $10^5$ Zellen/ml eingestellt. 160 ml dieser Suspension werden jeweils pro Well in Mikrotiterplatten pipettiert. Als Pseudoantigen werden 10 ml einer Lösung von HFC$\mu$S-IgG (300 $\mu$g/ml) zugegeben und als Wachstumsfaktor 20 $\mu$l BCGF (Cellular Products Incorporated) hinzugefügt. Dazu werden 20 $\mu$l der zu testenden Verbindung in 10facher Konzentration pipettiert. Die Kulturen werden insgesamt 140 h bei 37°, 5 % $CO_2$ und 95 % relativen Luftfeuchtigkeit inkubiert. 16 h vor Ablauf der Inkubationszeit wird jede Kultur mit 1 $\mu$ Ci einer [$^3$H]-Thymidin-Lösung gepulst. Am Ende des Versuches werden die Zellen mit einem Harvester geerntet und die eingebaute Radioaktivität in einem Flüssigszintillationszähler bestimmt.

Beispiel 2

Concanavalin A (ConA)-induzierte Proliferation muriner Splenozyten

Milzzellen (4 x $10^5$) von $CB6F_1$-Mäusen werden mit 0,5 $\mu$g/ml ConA in Mikrotiterplatten (Nunc GmbH, Wiesbaden, FRG) und verschiedenen Konzentrationen von Imexon in 6-fach Ansätzen für insgesamt 48 h inkubiert. 5 h vor Ablauf der Inkubationszeit werden die Kulturen mit [$^3$H]-Thymidin gepulst und anschlie-

ßend auf Glasfiberfilterplattchen mittels eines Multisample Harvester (Skatron A.S., Lier, Norwegen) geerntet. Die Filterplättchen werden getrocknet, und die Radioaktivität in einem Packard Szintillationsspektrometer bestimmt.

Beispiel 3

Phythämagglutinin (PHA)-induzierte Proliferation humaner Lymphozyten

1 ml humanes Vollblut wird mit 500 µg PHA-Lösung (500 µg/ml) und 48 ml DMEM-Mediun verdünnt. Jeweils 200 µl dieses Ansatzes werden mit 20 µl der zu testenden Imexonkonzentration in 6-fach-Ansätzen gemischt und 4 Tage inkubiert. Nach Pulsen mit [$^3$H]-Thymidin wird weitere 24 h inkubiert, geerntet und ausgewertet wie bei Beispiel 2 beschrieben.

Beispiel 4

Tumorwachstumsinhibitionsassay (TGI)

Die Methylcholanthren-induzierte Fibrosarkomzellinie (MethA) wurde aus der eigenen Tumorzellbank erhalten und intraperitoneal in $CB6F_1$-Mäusen passagiert.

1 x $10^4$ MethA-Zellen werden mit der zu testenden Imexonkonzentration in DMEM-Medium über 48 h inkubiert. 3 h vor Ablauf der Inkubationszeit wird mit [$^3$H]-Thymidin gepulst, geerntet und ausgewertet wie bei Beispiel 2 beschrieben.

Die in der Tabelle 1 angebenen Werte zeigen das Ergebnis eines repräsentativen Experimentes. Es sind die Ergebnisse der Untersuchungen mit Imexon im TGI-, LTT (ConA, PHA)- sowie im BCGF-Assay, d.h. der Einfluß von Imexon auf die MethA-Sarkomzell-, T-Lymphozyten- und B-Zell-Proliferation, dargestellt. Imexon supprimiert signifikant und spezifisch die BCGF-induzierte B-Zellproliferation bei einer Konzentration von 1µg/ml, wohingegen die Lymphozytenproliferation, induziert entweder durch ConA oder PHA, erst bei Konzentrationen > 10µg/ml signifikant gehemmt werden. Ferner ist die Spontanproliferation von MethA-Sarkomzellen ebenfalls erst ab > 10µg/ml signifikant unterdrückt.

Die Ergebnisse der obigen Versuche sind in der folgenden Tabelle 1 zusammengefaßt:

Tab. 1: Effekt von Imexon auf die Proliferation verschiedener Zelltypen

| Imexon (µg/ml) | TGI (MethA) $^3$H-TdR cpm (n=6) $\bar{x}$ | SD | %Hemmung | LTT (Splenozyten, ConA) $^3$H-TdR cpm (n=6) $\bar{x}$ | SD | %Hemmung | LTT (Splenozyten, PHA) $^3$H-TdR cpm (n=6) $\bar{x}$ | SD | %Hemmung | BCGF (humane B-Lymphozyten) $^3$H-TdR cpm (n=6) $\bar{x}$ | SD | %Hemmung |
|---|---|---|---|---|---|---|---|---|---|---|---|---|
| Kontrolle | 33966 (n=5) | 3000 | - | 109879 | 12203 | - | 44283 | 6458 | - | 5541 | 1792 | - |
| 100 | 534 | 363 | 98** | 903 | 62 | 99** | 585 | 44 | 99** | 562 | 44 | 90** |
| 30 | 911 | 110 | 97** | 2509 | 863 | 98** | 573 | 59 | 99** | 617 | 59 | 89** |
| 10 | 21913 | 2357 | 35** | 24895 | 6563 | 77** | 4724 | 704 | 89** | 574 | 50 | 90** |
| 3 | 35473 | 3135 | -4 | 118487 | 9494 | -8 | 35850 | 13018 | 19 | 831 | 231 | 85** |
| 1 | 35475 | 1753 | -4 | 119120 | 9172 | -8 | 49348 | 4168 | -11 | 2096 | 455 | 62* |
| 0.3 | 37593 | 3080 | -11 | 134032 | 37682 | -22 | 45542 | 9870 | -3 | 4201 | 1636 | 24 |
| 0.1 | 31722 | 3991 | 7 | 109717 | 11192 | 0 | 41849 | 1892 | 5 | 4847 | 1146 | 13 |

* $p < 0.002$

** $p < 0.001$

Beispiel 5

Wirkung von Imexon bei Autoimmunerkrankungen

Der Mäusestamm MRL 1pr/1pr entwickelt mit dem Lebensalter zunehmend spontan Lymphadenome und SLE-ähnliche Symptome, z. B. Synthese von antinukleâren Autoantikörpern. Zur Untersuchung des prophylaktischen Effektes von Imexon auf die Entwicklung dieser Symptome wurden 11 Wochen alte MRL Mäuse einmal täglich intraperitoneal mit den angegebenen Dosierungen Imexon und Cyclophosphamid behandelt. Dokumentiert wurden die Zahl der Lymphadenome und die Konzentration antinukleärer Antikörper. Bei der Untersuchung zur therapeutischen Potenz von Imexon wurden MRL Mäuse, nachdem jedes Tier mindestens ein Lymphadenom entwickelt hat (ca. 14 Wochen alte Tiere), ebenfalls einmal täglich mit den angegebenen Dosierungen an Imexon und Cyclophosphamid behandelt. Die Meßparameter waren wiederum Zahl der Lymphadenome sowie Autoantikörpertiter.

Die Ergebnisse dieser Untersuchungen haben gezeigt, daß Imexon bei sehr guter Verträglichkeit die Zahl spontan entstehender Lymphadenome und die Konzentration DNA spezifischer Antikörper erniedrigt. Auch beim therapeutischen Ansatz mit bereits lymphomtragenden Tieren zeigt sich die Wirksamkeit von Imexon. Die Zahl der Lymphadenome nimmt dosisabhängig ab, ebenso der Titer der Autoantikörper.

Beispiel 6

Herstellung einer pharmazeutischen Formulierung von Imexon

Als geeignete Arzneimittelzubereitung hat sich eine Filmtablette mit beispielsweise 100 mg Wirkstoff erwiesen, die wie folgt zusammengesetzt ist:

| | Gewicht/Stück/mg |
|---|---|
| Imexon | 100.000 |
| Lactose . 1$H_2O$ | 63.000 |
| Poly (O-carboxymethyl)stärke, Na-Salz | 7.000 |
| Poly (1-vinyl-2-pyrrolidon) 25.000 | 4.000 |
| Poly (O-carboxymethyl)stärke, Na-Salz | 3.000 |
| MIkrokristalline Cellulose | 20.000 |
| Siliciumdioxid, hochdispers | 1.500 |
| Magnesiumstearat | 1.500 |
| Kerngewicht: | 200.000 |

Die Filmtabletten werden dann durch Filmdragierung der erhaltenen Imexonkerne in üblicher Weise hergestellt.

Filmtabletten mit z.B. 10 mg, 50 mg, 200 mg oder 500 mg Wirkstoff werden in entsprechender Weise hergestellt.

**Patentansprüche**

1. Verwendung von Imexon zur Herstellung von Arzneimitteln mit immunsuppressiver Wirkung zur Behandlung von Krankheiten, die mit einer erhöhten B-Lymphozyten-Aktivierung verbunden sind.

2. Verwendung von Imexon gemäß Anspruch 1 zur Herstellung von Arzneimitteln zur Behandlung von Autoimmunerkrankungen.

3. Verwendung gemäß einem der Ansprüche 1 oder 2, dadurch gekennzeichnet, daß Imexon in einer Menge von 10 bis 1000 mg / Applikationsform eingesetzt wird.

4. Verwendung gemäß einem der Ansprüche 1 oder 2, dadurch gekennzeichnet, daß Imexon in Kombination mit einem weiteren immunsuppressiven Wirkstoff eingesetzt wird.

5. Verwendung gemäß Anspruch 1, dadurch gekennzeichnet, daß Imexon in Kombination mit einem antiretroviralen Wirkstoff eingesetzt wird.

## Claims

1. Use of imexon for the production of medicaments with immunosuppressive action for the treatment of diseases which are involved with an increased B-lymphocyte activation.

2. Use of imexon according to claim 1 for the production of medicaments for the treatment of autoimmune diseases.

3. Use according to one of claims 1 or 2, characterised in that imexon is used in an amount of 10 to 1000 mg/form of administration.

4. Use according to one of claims 1 or 2, characterised in that imexon is used in combination with a further immunosuppressive active material.

5. Use according to claim 1, characterised in that imexon is used in combination with an anti-retroviral active material.

## Revendications

1. Utilisation de l'imexone pour la préparation de médicaments ayant un effet immunosuppresseur, pour le traitement de maladies liées à une activation accrue des lymphocytes B.

2. Utilisation de l'imexone selon la revendication 1, pour la préparation de médicaments destinés au traitement de maladies autoimmunes.

3. Utilisation selon l'une quelconque des revendications 1 ou 2, caractérisée en ce que l'imexone est utilisée en une quantité de 10 à 1000 mg/forme galénique.

4. Utilisation selon l'une quelconque des revendications 1 ou 2, caractérisée en ce que l'imexone est utilisée en combinaison avec une autre substance active immunosuppressive.

5. Utilisation de l'imexone selon la revendication 1, caractérisée en ce que l'imexone est utilisée en combinaison avec une substance active antirétrovirale.